# EUROPEAN PATENT APPLICATION

(11) **EP 2 090 561 A1**
(43) Date of publication of application: **19.08.2009**
(21) Application number: 08151146.1
(22) Date of filing: 07.02.2008
(51) Int. Cl.: C07C 1/24, C07C 11/04, C07C 11/06, C07C 11/08, C07C 15/46

(54) **Dehydration of alcohols on crystalline silicates**

(71) Applicant: Total Petrochemicals Research Feluy, 7181 Seneffe (Feluy) (BE)
(72) Inventor: Minoux, Delphine, B-7181, Familleureux (BE); Nesterenko, Nikolai, B-1400, Nivelles (BE); Vermeiren, Walter, B -3530, Houthalen (BE); Van Donk, Sander, B-1180, Uccle (BE); Dath, Jean-Pierre, B-7970 Beloeil (BE)

(57) **Abstract**

The present invention is a process for the dehydratation of at least an alcohol to make at least an olefin, comprising:
introducing in a reactor a stream (A) comprising at least an alcohol, optionally water, optionally an inert component, contacting said stream with a catalyst in said reactor at conditions effective to dehydrate at least a portion of the alcohol to make an olefin,
recovering from said reactor an olefin containing stream (B), Wherein,
the catalyst is a crystalline silicate having a ratio Si/Al of at least about 100,
the WHSV on the alcohols is at least 4 h⁻¹,
the temperature ranges from 280°C to 450°C.
The invention is of interest for ethanol, propanol, butanol and phenylethanol, preferably for ethanol.
The crystalline silicate is advantageously selected among the MFI and the MEL type zeolites and the Si/Al ratio ranges from 100 to 1000.

## Description

### [Field of the invention]

The present invention relates to the dehydratation of at least an alcohol on crystalline silicates to make at least an olefin. The limited supply and increasing cost of crude oil has prompted the search for alternative processes for producing hydrocarbon products such as ethylene. Ethanol can be obtained by fermentation of carbohydrates. Made up of organic matter from living organisms, biomass is the world's leading renewable energy source.

### [Background of the invention]

US 4207424 describes a process for the catalytic dehydration of alcohols to form unsaturated organic compounds in which an alcohol is dehydrated in the presence of alumina catalysts which are pre-treated with an organic silylating agent at elevated temperature. Example 12 relates to ethanol, the WHSV is 1.2 h⁻¹ and shows only a conversion increase by comparison with the same alumina but having not been pretreated.

US 4302357 relates to an activated alumina catalyst employed in a process for the production of ethylene from ethanol through a dehydration reaction. In the description LHSV of ethanol is from 0.25 to 5 h⁻¹ and preferably from 0.5 to 3 h⁻¹. The examples are carried out at 370°C and LHSV of h⁻¹, ethylene yield is from 65 to 94%.

Process Economics Reviews PEP' 79-3 (SRI international) of december 1979 describes the dehydratation of an ethanol-water (95/5 weight %) mixture on a silica-alumina catalyst in a tubular fixed bed at 315-360°C, 1.7 bar absolute and a WHSV (on ethanol) of 0.3 h⁻¹. The ethanol conversion is 99% and the ethylene selectivity is 94.95%. It also describes the dehydratation of an ethanol-water (95/5 weight %) mixture on a silica-alumina catalyst in a fluidized bed at 399°C, 1.7 bar absolute and a WHSV (on ethanol) of 0.7 h⁻¹. The ethanol conversion is 99.6% and the ethylene selectivity is 99.3%.

US 4232179 relates to the preparation of ethylene, based on a process for dehydrating ethyl alcohol. More particularly, the object of said prior art is the production of ethylene in the presence of catalysts, using adiabatic reactors and a high temperature. Such adiabatic reactors may be used in parallel or may be arranged in series or arranged in assemblies of parallel series, or still only a single reactor may be used. The ratio between the sensible heat carrying stream and the feed may range from 0.2:1 to 20:1, but preferably shall be comprised within the range from 0.2:1 to 10:1. On the other hand the space velocity may range between 10 and 0.01 g/h of ethyl alcohol per gram of catalyst, depending on the desired operation severity, the range between 1.0 and 0.01 g/h/g being particularly preferred. In the examples the catalysts are silica alumina, the WHSV on ethanol is from 0.07 to 0.7, the ratio of steam to ethanol is from 3 to 5.

EP 22640 relates to improved zeolite catalysts, to methods of producing such catalysts, and to their use in the conversion of ethanol and ethylene to liquid and aromatic hydrocarbons, including the conversion of ethanol to ethylene. More particularly this prior art relates to the use of zeolite catalysts of Si/Al ratio from 11 to 24 (in the examples) such as the ZSM and related types in the conversion reaction of aqueous and anhydrous ethanol to ethylene, of aqueous ethanol to higher hydrocarbons, and of ethylene into liquid and aromatic hydrocarbons. WHSV ranges from 5.3 to 6 h⁻¹, in dehydratation to ethylene the reactor temperature is from 240 to 290°C.

US 4727214 relates to a process for converting anhydrous or aqueous ethanol into ethylene wherein at least one catalyst of the crystalline zeolite type is used, said catalyst having, on the one hand, channels or pores formed by cycles or rings of oxygen atoms having 8 and/or 10 elements or members. In the examples the atomic ratio Si/Al is from 2 to 45, the temperature from 217 to 400°C and the WHSV 2.5 h⁻¹.

US 4847223 describes a catalyst comprising from 0.5 to 7% by weight of trifluoromethanesulfonic acid incorporated onto an acid-form pentasil zeolite having a Si/Al atomic ratio ranging from 5 to 54 and a process for producing same. Also within the scope of said prior art is a process for the conversion of dilute aqueous ethanol to ethylene comprising: flowing said ethanol through a catalyst comprising from 0.5 to 7% by weight of trifluoromethanesulfonic acid incorporated onto an acid-form pentasil zeolite having a Si/Al atomic ratio range from 5 to 54 at a temperature ranging from 170° to 225° C and recovering the desired product. The WHSV is from 1 to 4.5 h⁻¹. The zeolites which are directly concerned by said prior art belong to the family called ZSM or pentasil zeolite family namely ZSM-5 and ZSM-11 type zeolites.

US 4873392 describes a process for converting diluted ethanol to ethylene which comprises heating an ethanol-containing fermentation broth thereby to vaporize a mixture of ethanol and water and contacting said vaporized mixture with a ZSM-5 zeolite catalyst selected from the group consisting of :
- a ZSM-5 zeolite having a Si/Al atomic ratio of from 5 to 75 which has been treated with steam at a temperature ranging from 400 to 800°C for a period of from 1 to 48 hours;
- a ZSM-5 zeolite having a Si/Al atomic ratio of from 5 to 50 and wherein La or Ce ions have been incorporated in a weight percentage of 0.1 to 1.0% by ion exchange or in a weight percentage ranging from 0.1 to 5% by impregnation, and
- a ZSM-5 zeolite having a Si/Al of from 5 to 50 and impregnated with a 0.5 to 7 wt % of trifluoromethanesulfonic acid,
and recovering the ethylene thus produced.

In ex 1 the catalyst is a steamed ZSM-5 having a Si/Al ratio of 21, the aqueous feed contains 10 w% of ethanol and 2 w% of glucose, the temperature is 275°C, the WHSV is from 3.2 to 38.5 h⁻¹. The ethylene yield decreases with the increase of WHSV. The ethylene yield is 99.4% when WHSV is 3.2 h⁻¹ and 20.1% when WHSV is 38.5 h⁻¹.

In ex 2 a ZSM-5 having a Si/Al ratio of 10 is compared with the same but on which La or Ce ions have been incorporated. The aqueous feed contains 10 w% of ethanol and 2 w% of glucose, the temperature is from 200°C to 225°C, the WHSV is 1 h⁻¹ and the best ethylene yield is 94.9%.

In ex 3 the catalyst is a ZSM-5 having a Si/Al ratio of 10 on which trifluoromethanesulfonic acid has been incorporated, the aqueous feed contains 10 w% of ethanol and 2 w% of glucose, the temperature is from 180°C to 205°C, the WHSV is 1 h⁻¹. The ethylene yield increases with temperature (73.3% at 180°C, 97.2% at 200°C) and then decreases (95.8% at 205°C).

US 4670620 describes ethanol dehydratation to ethylene on ZSM-5 catalysts. In a preferred embodiment the catalysts used according to this prior art are of the ZSM-5 type and preferably at least partially under hydrogen form. In the examples the catalyst is a ZSM-5 or a ZSM-11 having a SI/Al ratio of 40 to 5000 (ex 13), the LHSV is from 0.1 to 0.31 h⁻¹ and the temperature from 230°C to 415°C.

It has now been discovered that the dehydratation of at least an alcohol to at least an olefin can be made on a crystalline silicate having a high Si/Al ratio and with a WHSV of at least 4 h⁻¹.

By way of example, in the dehydratation of ethanol to make ethylene, the ethanol conversion is at least 99%, the ethylene yield is at least 97%, the ethylene selectivity is at least 97% and the ethylene purity is at least 99.8%. The ethanol conversion is the ratio (ethanol introduced in the reactor - ethanol leaving the reactor)/ (ethanol introduced in the reactor).
The ethylene yield is the ratio, on carbon basis, (ethylene leaving the reactor)/ (ethanol introduced in the reactor).
The ethylene selectivity is the ratio, on carbon basis, (ethylene leaving the reactor)/ (ethanol converted in the reactor).
The ethylene purity is the ratio, on carbon basis, (ethylene leaving the reactor)/ (ethylene + ethane leaving the reactor). It means the ethylene purity is the percentage of ethylene, on a carbon basis, present in the C₂ cut recovered in the stream leaving the reactor. The C₂ cut doesn't comprise the unconverted ethanol and acetaldehyde if any. The same definitions apply mutatis mutandis to the alcohol and the olefin.

### [Brief summary of the invention]

The present invention relates to a process for the dehydratation of at least an alcohol to make at least an olefin, comprising :
introducing in a reactor a stream (A) comprising at least an alcohol, optionally water, optionally an inert component, contacting said stream with a catalyst in said reactor at conditions effective to dehydrate at least a portion of the alcohol to make an olefin,
recovering from said reactor an olefin containing stream (B), Wherein,
the catalyst is a crystalline silicate having a ratio Si/Al of at least about 100,
the WHSV on the alcohols is at least 4 h⁻¹,
the temperature ranges from 280°C to 450°C.

### [Detailed description of the invention]

**As regards the stream (A)**, The alcohol is any alcohol provided it can be dehydrated to the corresponding olefin. By way of example mention may be made of alcohols having from 2 to 10 carbon atoms. Advantageously the invention is of interest for ethanol, propanol, butanol and phenylethanol.

The inert component is any component provided there is no adverse effect on the catalyst. Because the dehydratation is endothermic the inert component can be used to bring energy. By way of examples the inert component is selected among the saturated hydrocarbon having up to 8 carbon atoms, nitrogen and carbon dioxide. Advantageously the inert component is a saturated hydrocarbon having from 3 to 6 carbon atoms and is preferably pentane. The weight proportions of respectively alcohol, water and inert component are, for example, 10-100/0-20/0-70 (the total being 100). The stream (A) can be liquid or gaseous.

**As regards the reactor**, it can be a fixed bed reactor, a moving bed reactor or a fluidized bed reactor. A typical fluid bed reactor is one of the FCC type used for fluidized-bed catalytic cracking in the oil refinery. A typical moving bed reactor is of the continuous catalytic reforming type. The dehydratation may be performed continuously using a pair of parallel "swing" reactors. The various preferred catalysts of the present invention have been found to exhibit high stability. This enables the dehydratation process to be performed continuously in two parallel "swing" reactors wherein when one reactor is operating, the other reactor is undergoing catalyst regeneration. The catalyst of the present invention also can be regenerated several times.

**As regards the pressure,** it can be any pressure but it is more easy and economical to operate at moderate pressure. By way of example the pressure of the reactor ranges from 0.5 to 5 bars absolute (50 kPa to 0.5 MPa) and advantageously from 1.2 to 4 bars absolute (0.12 MPa to 0.4 MPa).

**As regards the temperature**, it ranges from 280°C to 450°C, advantageously from 300°C to 400°C and preferably from 330°C to 380°C.

**As regards the WHSV,** it ranges advantageously from 5 to 15 h⁻¹, preferably from 7 to 12 h⁻¹.

**As regards the stream (B),** it comprises essentially water, olefin, the inert component (if any) and unconverted alcohol. Said unconverted alcohol is supposed to be as less as possible. The olefin is recovered by usual fractionation means. Advantageously the inert component, if any, is recycled in the stream (A) as well as the unconverted alcohol, if any. Unconverted alcohol, if any, is recycled to the reactor in the stream (A).

**As regards the crystalline silicate,** it is by way of example of the MFI or MEL type, the three-letter designations "MFI" and "MEL" each representing a particular crystalline silicate structure type as established by the Structure Commission of the International Zeolite Association. Examples of a crystalline silicate of the MFI type are the synthetic zeolite ZSM-5 and silicalite and other MFI type crystalline silicates known in the art. Examples of a crystalline silicate of the MEL family are the zeolite ZSM-11 and other MEL type crystalline silicates known in the art. Other examples are Boralite D and silicalite-2 as described by the International Zeolite Association (Atlas of zeolite structure types, 1987, Butterworths). The preferred crystalline silicates have pores or channels defined by ten oxygen rings and a high silicon/aluminium atomic ratio.

Crystalline silicates are microporous crystalline inorganic polymers based on a framework of XO₄ tetrahedra linked to each other by sharing of oxygen ions, where X may be trivalent (*e.g*. Al,B,...) or tetravalent (*e.g*. Ge, Si,...). The crystal structure of a crystalline silicate is defined by the specific order in which a network of tetrahedral units are linked together. The size of the crystalline silicate pore openings is determined by the number of tetrahedral units, or, alternatively, oxygen atoms, required to form the pores and the nature of the cations that are present in the pores. They possess a unique combination of the following properties: high internal surface area; uniform pores with one or more discrete sizes; ion exchangeability; good thermal stability; and ability to adsorb organic compounds. Since the pores of these crystalline silicates are similar in size to many organic molecules of practical interest, they control the ingress and egress of reactants and products, resulting in particular selectivity in catalytic reactions. Crystalline silicates with the MFI structure possess a bidirectional intersecting pore system with the following pore diameters: a straight channel along [010]:0.53-0.56 nm and a sinusoidal channel along [100]:0.51-0.55 nm. Crystalline silicates with the MEL structure possess a bidirectional intersecting straight pore system with straight channels along [100] having pore diameters of 0.53-0.54 nm.

In this specification, the term "silicon/aluminium atomic ratio" or "silicon/aluminium ratio" is intended to mean the Si/Al atomic ratio of the overall material, which may be determined by chemical analysis. In particular, for crystalline silicate materials, the stated Si/Al ratios apply not just to the Si/Al framework of the crystalline silicate but rather to the whole material.

The catalyst preferably has a high silicon/aluminium atomic ratio, of at least about 100, preferably greater than about 150, more preferably greater than about 200, whereby the catalyst has relatively low acidity. The acidity of the catalyst can be determined by the amount of residual ammonia on the catalyst following contact of the catalyst with ammonia which adsorbs to the acid sites on the catalyst with subsequent ammonium desorption at elevated temperature measured by differential thermogravimetric analysis. Preferably, the silicon/aluminium ratio (Si/Al) ranges from about 100 to about 1000, most preferably from about 200 to about 1000. Such catalysts are known per se. "about 100" means that 100 is not a strict ratio but correponds to a crystalline silicate having an acidity low enough to prevent high catalytic activity in addition to the dehydratation to olefin. At a Si/Al ratio above about 100 there is essentially a dehydratation to olefin and almost no side reactions which could lead to aldehydes, to saturated hydrocarbons or any undesirable component.

In a specific embodiment the crystalline silicate is steamed to remove aluminium from the crystalline silicate framework. The steam treatment is conducted at elevated temperature, preferably in the range of from 425 to 870°C, more preferably in the range of from 540 to 815°C and at atmospheric pressure and at a water partial pressure of from 13 to 200kPa. Preferably, the steam treatment is conducted in an atmosphere comprising from 5 to 100% steam. The steam atmosphere preferably contains from 5 to 100vol% steam with from 0 to 95vol% of an inert gas, preferably nitrogen. A more preferred atmosphere comprises 72 vol% steam and 28 vol% nitrogen i.e. 72kPa steam at a pressure of one atmosphere. The steam treatment is preferably carried out for a period of from 1 to 200 hours, more preferably from 20 hours to 100 hours. As stated above, the steam treatment tends to reduce the amount of tetrahedral aluminium in the crystalline silicate framework, by forming alumina.

In a more specific embodiment the crystalline silicate catalyst is dealuminated by heating the catalyst in steam to remove aluminium from the crystalline silicate framework and extracting aluminium from the catalyst by contacting the catalyst with a complexing agent for aluminium to remove from pores of the framework alumina deposited therein during the steaming step thereby to increase the silicon/aluminium atomic ratio of the catalyst. The catalyst having a high silicon/aluminium atomic ratio for use in the catalytic process of the present invention is manufactured by removing aluminium from a commercially available crystalline silicate. By way of example a typical commercially available silicalite has a silicon/aluminium atomic ratio of around 120. In accordance with the present invention, the commercially available crystalline silicate is modified by a steaming process which reduces the tetrahedral aluminium in the crystalline silicate framework and converts the aluminium atoms into octahedral aluminium in the form of amorphous alumina. Although in the steaming step aluminium atoms are chemically removed from the crystalline silicate framework structure to form alumina particles, those particles cause partial obstruction of the pores or channels in the framework. This could inhibit the dehydratation process of the present invention. Accordingly, following the steaming step, the crystalline silicate is subjected to an extraction step wherein amorphous alumina is removed from the pores and the micropore volume is, at least partially, recovered. The physical removal, by a leaching step, of the amorphous alumina from the pores by the formation of a water-soluble aluminium complex yields the overall effect of de-alumination of the crystalline silicate. In this way by removing aluminium from the crystalline silicate framework and then removing alumina formed therefrom from the pores, the process aims at achieving a substantially homogeneous de-alumination throughout the whole pore surfaces of the catalyst. This reduces the acidity of the catalyst. The reduction of acidity ideally occurs substantially homogeneously throughout the pores defined in the crystalline silicate framework. Following the steam treatment, the extraction process is performed in order to de-aluminate the catalyst by leaching. The aluminium is preferably extracted from the crystalline silicate by a complexing agent which tends to form a soluble complex with alumina. The complexing agent is preferably in an aqueous solution thereof. The complexing agent may comprise an organic acid such as citric acid, formic acid, oxalic acid, tartaric acid, malonic acid, succinic acid, glutaric acid, adipic acid, maleic acid, phthalic acid, isophthalic acid, fumaric acid, nitrilotriacetic acid, hydroxyethylenediaminetriacetic acid, ethylenediaminetetracetic acid, trichloroacetic acid trifluoroacetic acid or a salt of such an acid (*e*.*g*. the sodium salt) or a mixture of two or more of such acids or salts. The complexing agent for aluminium preferably forms a water-soluble complex with aluminium, and in particular removes alumina which is formed during the steam treatment step from the crystalline silicate. A particularly preferred complexing agent may comprise an amine, preferably ethylene diamine tetraacetic acid (EDTA) or a salt thereof, in particular the sodium salt thereof. In a preferred embodiment, the framework silicon/aluminium ratio is increased by this process to a value of from about 150 to 1000, more preferably at least 200.

Following the aluminium leaching step, the crystalline silicate may be subsequently washed, for example with distilled water, and then dried, preferably at an elevated temperature, for example around 110°C.

Additionally, if during the preparation of the catalysts of the invention alkaline or alkaline earth metals have been used, the molecular sieve might be subjected to an ion-exchange step. Conventionally, ion-exchange is done in aqueous solutions using ammonium salts or inorganic acids.

Following the de-alumination step, the catalyst is thereafter calcined, for example at a temperature of from 400 to 800°C at atmospheric pressure for a period of from 1 to 10 hours.

In another specific embodiment the crystalline silicate catalyst is mixed with a binder, preferably an inorganic binder, and shaped to a desired shape, *e*.*g*. pellets. The binder is selected so as to be resistant to the temperature and other conditions employed in the dehydratation process of the invention. The binder is an inorganic material selected from clays, silica, metal oxides such as Zr0₂ and/or metals, or gels including mixtures of silica and metal oxides. The binder is preferably alumina-free. If the binder which is used in conjunction with the crystalline silicate is itself catalytically active, this may alter the conversion and/or the selectivity of the catalyst. Inactive materials for the binder may suitably serve as diluents to control the amount of conversion so that products can be obtained economically and orderly without employing other means for controlling the reaction rate. It is desirable to provide a catalyst having a good crush strength. This is because in commercial use, it is desirable to prevent the catalyst from breaking down into powder-like materials. Such clay or oxide binders have been employed normally only for the purpose of improving the crush strength of the catalyst. A particularly preferred binder for the catalyst of the present invention comprises silica. The relative proportions of the finely divided crystalline silicate material and the inorganic oxide matrix of the binder can vary widely. Typically, the binder content ranges from 5 to 95% by weight, more typically from 20 to 50% by weight, based on the weight of the composite catalyst. Such a mixture of crystalline silicate and an inorganic oxide binder is referred to as a formulated crystalline silicate. In mixing the catalyst with a binder, the catalyst may be formulated into pellets, extruded into other shapes, or formed into a spray-dried powder. Typically, the binder and the crystalline silicate catalyst are mixed together by an extrusion process. In such a process, the binder, for example silica, in the form of a gel is mixed with the crystalline silicate catalyst material and the resultant mixture is extruded into the desired shape, for example pellets. Thereafter, the formulated crystalline silicate is calcined in air or an inert gas, typically at a temperature of from 200 to 900°C for a period of from 1 to 48 hours. The binder preferably does not contain any aluminium compounds, such as alumina. This is because as mentioned above the preferred catalyst for use in the invention is de-aluminated to increase the silicon/aluminium ratio of the crystalline silicate. The presence of alumina in the binder yields other excess alumina if the binding step is performed prior to the aluminium extraction step. If the aluminium-containing binder is mixed with the crystalline silicate catalyst following aluminium extraction, this re-aluminates the catalyst.

In addition, the mixing of the catalyst with the binder may be carried out either before or after the steaming and extraction steps.

In another embodiment the catalyst is a crystalline silicate catalyst having a monoclinic structure, which has been produced by a process comprising providing a crystalline silicate of the MFI-type having a silicon/aluminium atomic ratio lower than 80; treating the crystalline silicate with steam and thereafter leaching aluminium from the zeolite by contact with an aqueous solution of a leachant to provide a silicon/aluminium atomic ratio in the catalyst of at least 180 whereby the catalyst has a monoclinic structure.

Preferably, in the steam treatment step the temperature is from 425 to 870°C, more preferably from 540 to 815°C, and at a water partial pressure of from 13 to 200kPa.

Preferably, the aluminium is removed by leaching to form an aqueous soluble compound by contacting the zeolite with an aqueous solution of a complexing agent for aluminium which tends to form a soluble complex with alumina.

In accordance with this preferred process for producing monoclinic crystalline silicate, the starting crystalline silicate catalyst of the MFI-type has an orthorhombic symmetry and a relatively low silicon/aluminium atomic ratio which can have been synthesized without any organic template molecule and the final crystalline silicate catalyst has a relatively high silicon/aluminium atomic ratio and monoclinic symmetry as a result of the successive steam treatment and aluminium removal. After the aluminium removal step, the crystalline silicate may be ion exchanged with ammonium ions. It is known in the art that such MFI-type crystalline silicates exhibiting orthorhombic symmetry are in the space group Pnma. The x-ray diffraction diagram of such an orthorhombic structure has one peak at d = around 0.365nm, d = around 0.305nm and d= around 0.300 nm (see EP-A-0146524).

The starting crystalline silicate has a silicon/aluminium atomic ratio lower than 80. A typical ZSM-5 catalyst has 3.08 wt% Al₂O₃, 0.062 wt% Na₂0, and is 100% orthorhombic. Such a catalyst has a silicon/aluminium atomic ratio of 26.9.

The steam treatment step is carried out as explained above. The steam treatment tends to reduce the amount of tetrahedral aluminium in the crystalline silicate framework by forming alumina. The aluminium leaching or extraction step is carried out as explained above. In the aluminium leaching step, the crystalline silicate is immersed in the acidic solution or a solution containing the complexing agent and is then preferably heated, for example heated by reflux, for an extended period of time, for example 18 hours. Following the aluminium leaching step, the crystalline silicate is subsequently washed, for example with distilled water, and then dried, preferably at an elevated temperature, for example around 110°C. Optionally, the crystalline silicate is subjected to ion exchange with ammonium ions, for example by immersing the crystalline silicate in an aqueous solution of NH₄Cl. Such an ion exchange step is desirable if the amount of sodium ions present in the crystalline silicate is so high that formation of the monoclinic crystalline form is prevented.

Finally, the catalyst is calcined at an elevated temperature, for example at a temperature of at least 400°C. The calcination period is typically around 3 hours.

The resultant crystalline silicate has monoclinic symmetry, being in the space group P2₁/n. The x-ray diffraction diagram of the monoclinic structure exhibits three doublets at d = around 0.36, 0.31 and 0.19nm. The presence of such doublets is unique for monoclinic symmetry. More particularly, the doublet at d = around 0.36, comprises two peaks, one at d = 0.362nm and one at d = 0.365nm. In contrast, the orthorhombic structure has a single peak at d = 0.365nm.

The presence of a monoclinic structure can be quantified by comparing the x-ray diffraction line intensity at d = around 0.36nm. When mixtures of MFI crystalline silicates with pure orthorhombic and pure monoclinic structure are prepared, the composition of the mixtures can be expressed as a monoclinicity index (in%). The x-ray diffraction patterns are recorded and the peak height at d=0.362nm for monoclinicity and d=0.365nm for orthorhombicity is measured and are denoted as Im and Io respectively. A linear regression line between the monoclinicity index and the Im/Io gives the relation needed to measure the monoclinicity of unknown samples. Thus the monoclinicity index % = (axlm/lob)x100, where a and b are regression parameters.

The monoclinic crystalline silicate can be produced having a relatively high silicon/aluminium atomic ratio of at least 180, preferably greater than about 200 without using an organic template module. Furthermore, the crystallite size of the monoclinic crystalline silicate can be kept relatively low, typically less than 1 micron, more typically around 0.5 microns, since the starting crystalline silicate has low crystallite size which is not increased by the subsequent process steps. Accordingly, since the crystallite size can be kept relatively small, this can yield a corresponding increase in the activity of the catalyst. This is an advantage over known monoclinic crystalline silicate catalysts where typically the crystallite size is greater than 1 micron.

One skilled in the art will also appreciate that the olefins made by the dehydratation process of the present invention can be, by way of example, polymerized. When the olefin is ethylene it can be, by way of example,
polymerized to form polyethylenes,
dimerized to butene and then to isobutene, said isobutene reacting with ethanol to produce ETBE,
converted to ethylene oxide and glycol or
converted to vinyl chloride.
The present invention relates also to said polyethylenes, butene, isobutene, ETBE, vinyl chloride, ethylene oxide and glycol.

### [Examples]

The stainless-steel reactor tube has an internal diameter of 10mm. 10ml of catalyst, as pellets of 35-45 mesh, is loaded in the tubular reactor. The void spaces before and after the catalyst are filled with SiC granulated of 2mm.The temperature profile is monitored with the aid of a thermocouple well placed inside the reactor. The reactor temperature is increased at a rate of 60°C/h to 550°C under air, kept 2 hours at 550°C and then purged by nitrogen. The nitrogen is then replaced by the feed (either a pure ethanol feed or an aqueous ethanol feed). The catalytic tests are then performed down-flow, at near atmospheric pressure (pressure of 1.35bara), in a temperature range of 300-450°C and with a weight hour space velocity (WHSV) varying from 2 to 10h⁻¹. Analysis of the products is performed by using an *on-line* gas chromatograph.

### Example 1 (comparative) : γ-Al₂O₃

A γ-Al₂O₃ as 1.5mm extrudates exhibits the following textural properties : a specific surface area of 285 m²/g, with a porous distribution centered around 94A, and a porous volume of 0.67 ml/g. The impurities present on the alumina in small amounts are summarized below:
0.51 %wt S, 0.4%wt Si, 0.04%wt Ca, 0.08%wt Cl, 0.02%wt Fe, 0.01 %wt Cu.

### Catalyst performances:

For the following experiments, a pure ethanol feed has been used.
■ At 400°C, under 1.35 bara and with an ethanol space velocity of 1.8h⁻¹, the ethanol conversion is almost complete (> 99.7%wt), with a C₂⁼ selectivity of 80%wt (CH₂ basis) and a C₂⁼ purity remaining above 98.2%wt.
The limited C₂⁼ selectivity can be explained by the fact that under these operating conditions, the formation of heavier compounds takes place, especially up to 12%wt (CH₂ basis) of C₄⁼ olefins and around 3%wt paraffins (CH₂ basis).

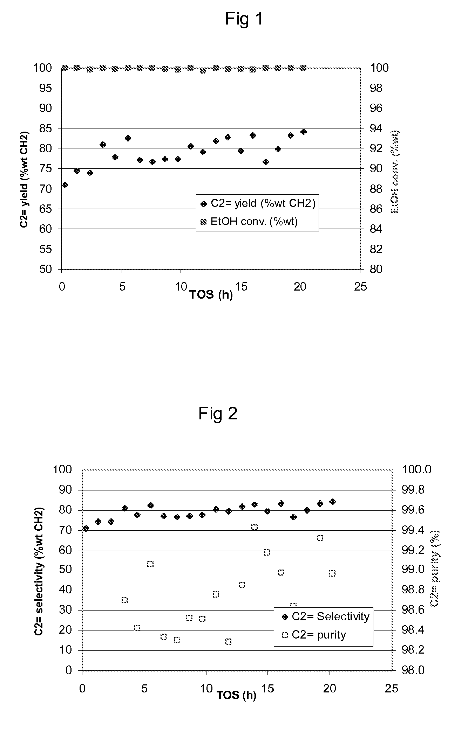

The results are displayed on fig1-3:
Figure1- Ethanol conversion and C₂⁼yield (wt CH₂ basis) as a function of TOS (Time On Stream) (h) ; 400°C-1.35bara-WHSV(EtOH)=1.8h⁻¹
Figure 2 - C₂⁼selectivity (wt CH₂ basis) and purity as a function of TOS (h) ; 400°C-1.35bara-WHSV(EtOH)=1.8h⁻¹
Figure 3 - C₄⁼ olefin and paraffin yield (wt CH₂ basis) as a function of TOS (h) ; 400°C-1.35bara-WHSV(EtOH)=1.8h⁻¹

The use of this γAl₂O₃ did not allow to reach good performances for ethylene dehydration. Without being binded by any explanation the inventors think it could be linked to the low purity of the alumina used (sulfur, silicon, iron contents especially are quite high).

### Example 2 (comparative) : Silica-Alumina

The silica-alumina under powder form exhibits a specific area of 377 m²/g, and consists in 94.4%wt Al₂O₃ and 5.6%wt SiO₂.

The catalyst has been first calcined at 600°C during 2 hours under air before being loaded.

### Catalyst performances:

For the following experiments, a pure ethanol feed has been used.
■ At 400°C, under 1.35 bara and with an ethanol space velocity of 2.8h⁻¹, the ethanol conversion is almost complete (> 99.9%wt), with a C₂⁼ selectivity of 90%wt (CH₂ basis) and a C₂⁼ purity remaining above 99%wt.
Compared to γ-Al₂O₃, better performances are achieved in terms of selectivity and purity. But this time again, the amount of C₄⁼ olefins remains quite high (∼6% wt (CH₂ basis) C₄⁼).

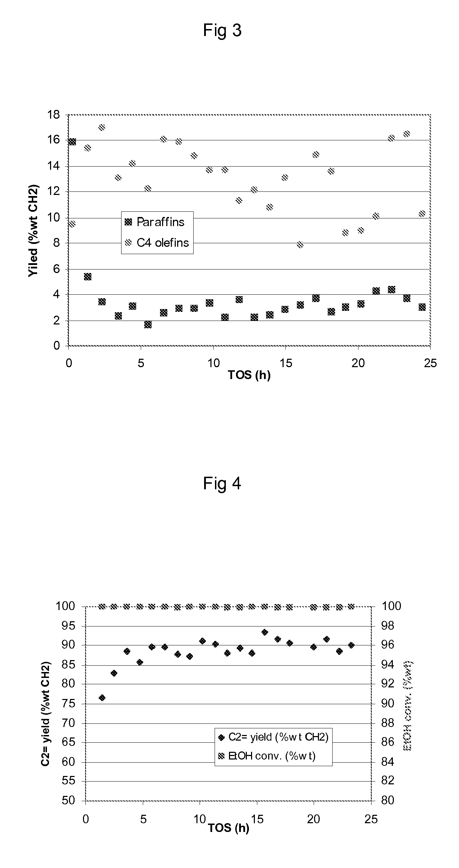

The results are displayed on fig 4-6
Figure 4- Ethanol conversion and C₂⁼ yield (wt CH₂ basis) as a function of TOS (h) ; 400°C-1.35bara-WHSV(EtOH)=2.8h⁻¹

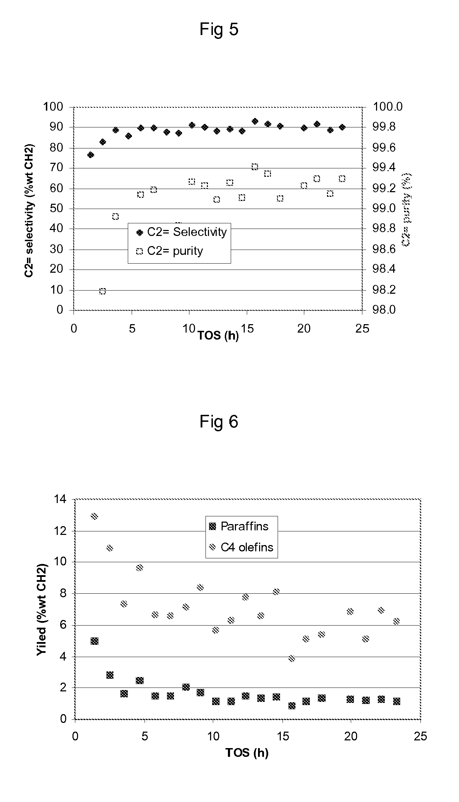

Figure 5 - C₂⁼ selectivity (wt CH₂ basis) and purity as a function of TOS (h) ; 400°C - 1.35bara -WHSV(EtOH)=2.8h⁻¹
Figure 6 - C4= olefin and paraffin yield (wt CH2 basis) as a function of TOS (h); 400°C-1.35bara-WHSV(EtOH)=2.8h⁻¹

■ Another set of operating conditions were then used in order to limit the formation of C₄⁺ coumpounds : the space velocity of ethanol was increased up to 5h⁻¹.

The C, H, N analysis of the spent catalyst at the end of the test reveals a carbon content of 5.2%wt indicating that coking occurs in that case also in quite a large extent, while simultaneously, the formation of heavies decreases slightly.

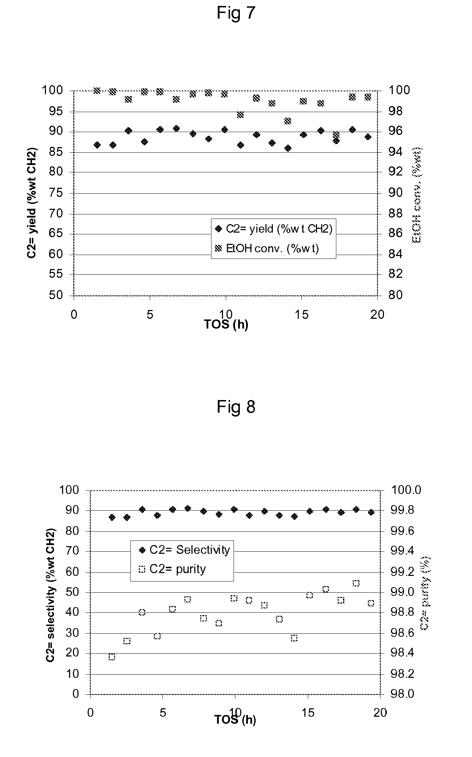

The results are displayed on Fig 7-9
Figure 7- Ethanol conversion and C2= yield (wt CH2 basis) as a function of TOS (h) ; 400°C-1.35bara-WHSV(EtOH)=5h⁻¹
Figure 8 - C2= selectivity (wt CH2 basis) and purity as a function of TOS (h) ; 400°C - 1.35bara -WHSV(EtOH)=5h⁻¹

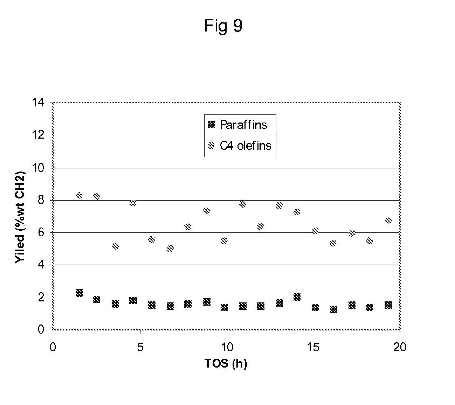

Figure 9 - C4= olefin and paraffin yield (wt CH2 basis) as a function of TOS (h) ; 400°C-1.35bara-WHSV(EtOH)=5h⁻¹

The use of a silica-alumina catalyst, allows to reach better catalytic performances for ethylene dehydration than gamma alumina. However, it is interesting to note that despite a quite important formation of heavy compounds (C₄⁺) at moderate space velocity (2.8h⁻¹), an increase of the ethanol flowrate does not allow to improve the catalytic performances towards ethylene dehydration : ethanol conversion is no more complete.

### Example 3 (according to the invention)

The silicalite used here is a zeolite H-ZSM-5 with a pure MFI structure having a Si/Al of 169 under powder form.

Catalyst performances: For the following experiments, a pure ethanol feed has been used at 350°C, under 1.35 bara and with an ethanol space velocity of up to 10h⁻¹.

In this set of operating conditions, ethanol conversion is almost complete (>98.8%wt), with a C₂⁼ selectivity of 96%wt (CH₂ basis) and a C₂⁼ purity remaining above 99.8%wt. Ethanol dehydration is the main reaction, as the temperature profile follow-up may testify it.

Very low amounts of C₄⁼, C₃⁼ and aromatics are now formed (0.9%wt, 0.7%wt and 0.2%wt (CH₂ basis) respectively).

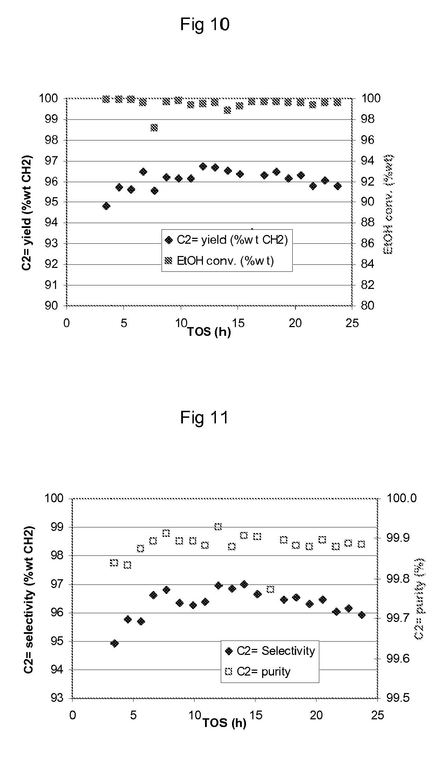

The results are displayed on Fig 10-12
Figure 10- Ethanol conversion and C2= yield (wt CH2 basis) as a function of TOS (h) ; 350°C-1.35bara-WHSV(EtOH)=10h⁻¹
Figure 11 - C2= selectivity (wt CH2 basis) and purity as a function of TOS (h) ; 350°C - 1.35bara -WHSV(EtOH)=10h⁻¹

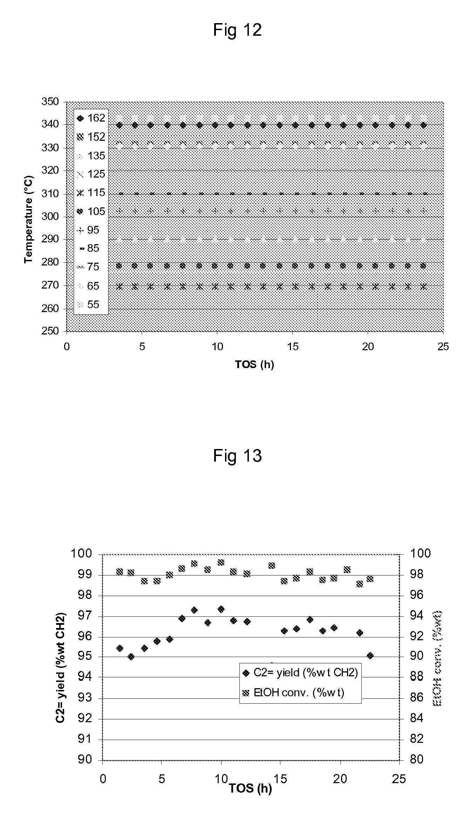

Figure 12 - Temperature profile along the catalytic bed as a function of TOS (h); 350°C-1.35bara-WHSV(EtOH)=10h⁻¹ the legend indicates the height at which the measurement is performed, the top of the bed being located at 162mm and the bottom of the bed corresponding to 55mm.

■ To check the impact of the feed, an aqueous ethanol has been used as a feed stock (95/5 %wt EtOH/H₂O).
The graphs reported below indicate that the presence of water even allows an improvement of C₂⁼ selectivity, reaching 98%wt (CH₂ basis), ethanol conversion being around 98%wt. A further decrease of temperature down to 300°C, did not allow to recover better results.

The results are displayed on fig 13-14
Figure13- Ethanol conversion and C2= yield (wt CH2 basis) as a function of TOS (h) ; 350°C-1.35bara-WHSV(EtOH)=10h⁻¹ (95/5) %wt EtOH/H2O mixture.

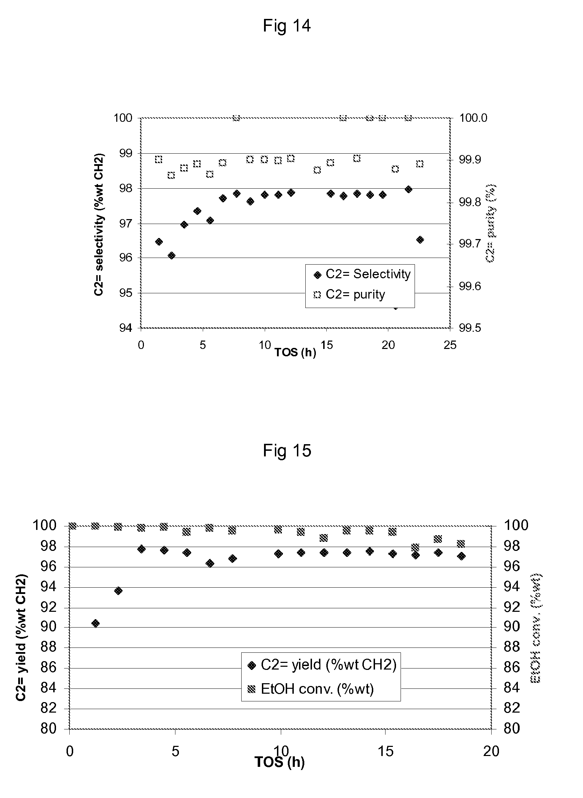

Figure 14 - C2= selectivity (wt CH2 basis) and purity as a function of TOS (h) ; 350°C - 1.35bara -WHSV(EtOH)=10h⁻¹ (95/5) %wt EtOH/H2O mixture.

The use of a high Si/Al silicalite allows to get very good catalytic performances for ethylene dehydration : ethanol conversion is almost complete (>98.8%wt), with a C₂⁼ selectivity of 96%wt (CH₂ basis) and a C₂⁼ purity remaining above 99.8%wt. It is worth also to underline that such results are obtained in a high space velocity range (10h⁻¹) : this could allow to increase the reactor throughput significantly. Furthermore, the use of aqueous ethanol (mixture of 95-5%wt EtOH-H₂O) leads to an improvement of C₂⁼ selectivity, though a slight decrease of ethanol conversion down to 98%wt, is noticed.

### Example 4 (according to the invention)

The catalyst is a shaped cylinder catalyst containing 30%wt of binder (silica) and 70%wt of silicalite (MFI), which has been steamed and acid exchanged, leading to an overall Si/Al of around 250.

Catalyst performances: By comparison with example 3 the operating conditions were set as follows : temperature was kept at 350°C, and the weight hourly space velocity decreased to 7h⁻¹, pressure being kept the same, and pure ethanol being used as the feed. In these operating conditions, ethanol conversion is very high (>97%wt), with a C₂⁼ selectivity of 98%wt (CH₂ basis) and a C₂⁼ purity remaining above 99.8%wt. Ethanol dehydration is the main reaction, as the temperature profile follow-up may testify it.

The results are displayed on fig 15-18
Figure 15- Ethanol conversion and C2= yield (wt CH2 basis) as a function of TOS (h) ; 350°C-1.35bara-WHSV(EtOH)=7h⁻¹

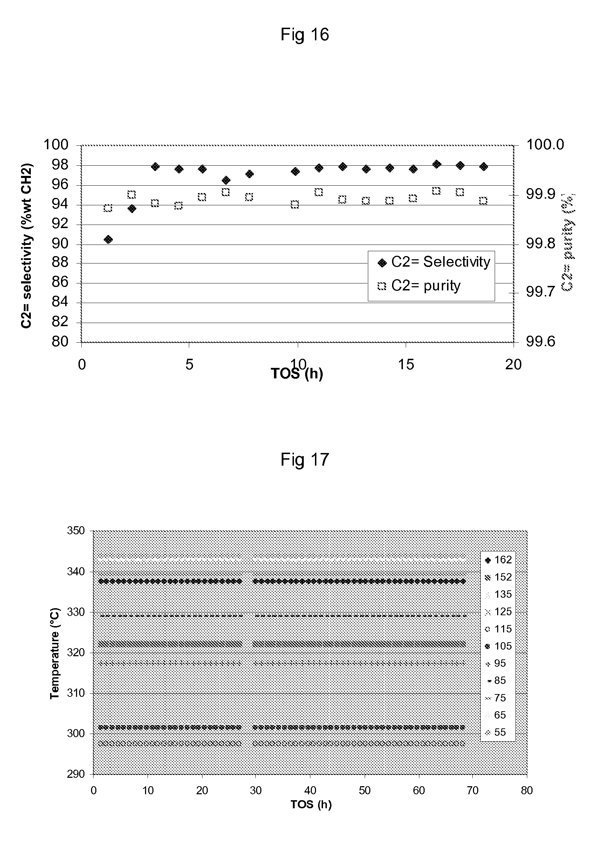

Figure 16 - C2= selectivity (wt CH2 basis) and purity as a function of TOS (h) ; 350°C - 1.35bara -WHSV(EtOH)=7h⁻¹
Figure 17 - Temperature profile along the catalytic bed as a function of TOS(h); 350°C- 1.35bara-WHSV(EtOH)=7h⁻¹ , the legend indicates the height at which the measurement is performed, the top of the bed being located at 162mm and the bottom of the bed corresponding to 55mm.

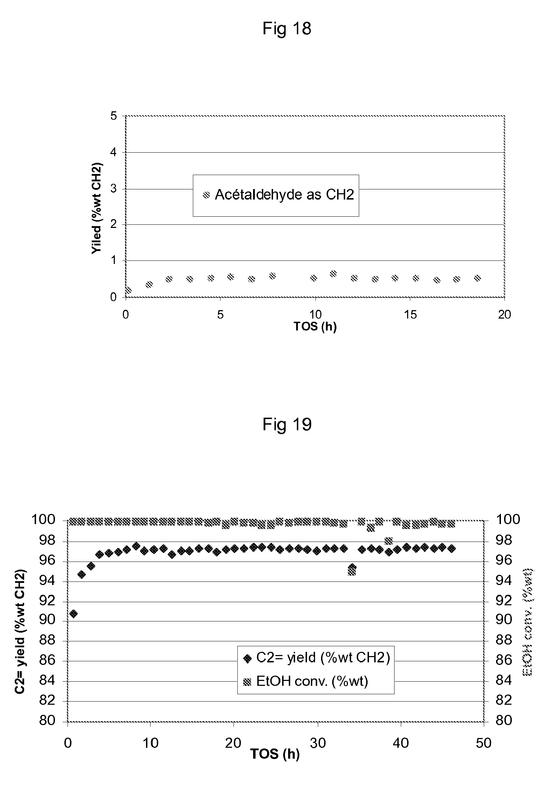

Figure 18 - acetaldehyde (wt CH2 basis) as a function of TOS (h) ; 350°C - 1.35bara -WHSV(EtOH)=7h⁻¹

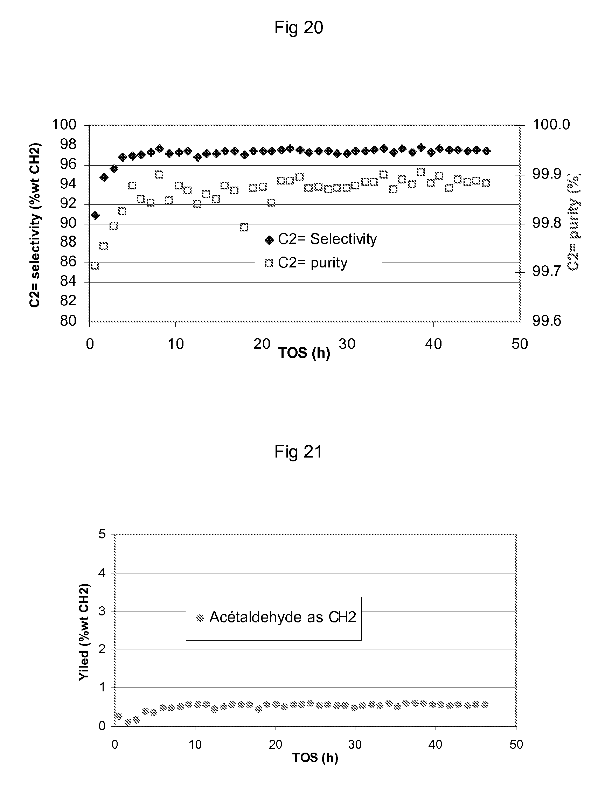

An other other set of dehydratations was carried out with the same conditions on the same catalyst but the WHSV is lowered to 5 h⁻¹ . The results are displayed on fig 19-22.

### Example 5 (comparative)

The catalyst is the same as in ex 4 : a shaped cylinder catalyst containing 30%wt of binder (silica) and 70%wt of silicalite (MFI), which has been steamed and acid exchanged, leading to an overall Si/Al of around 250.

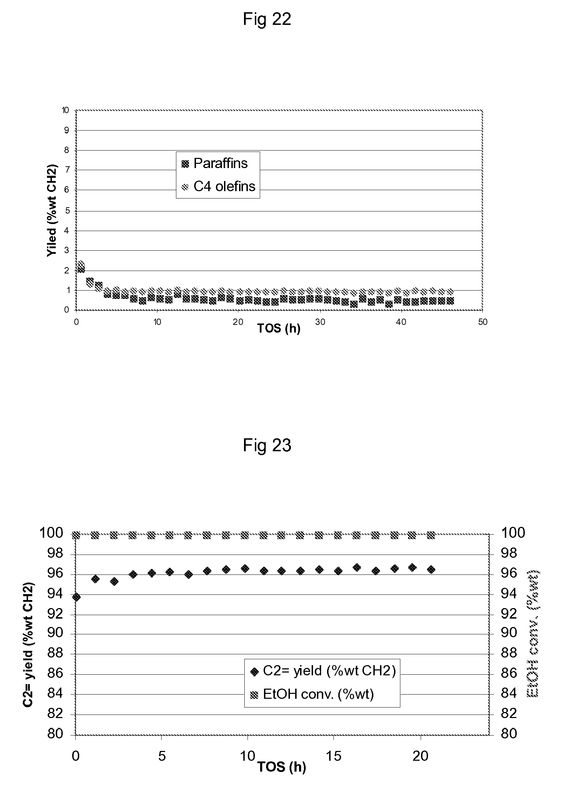

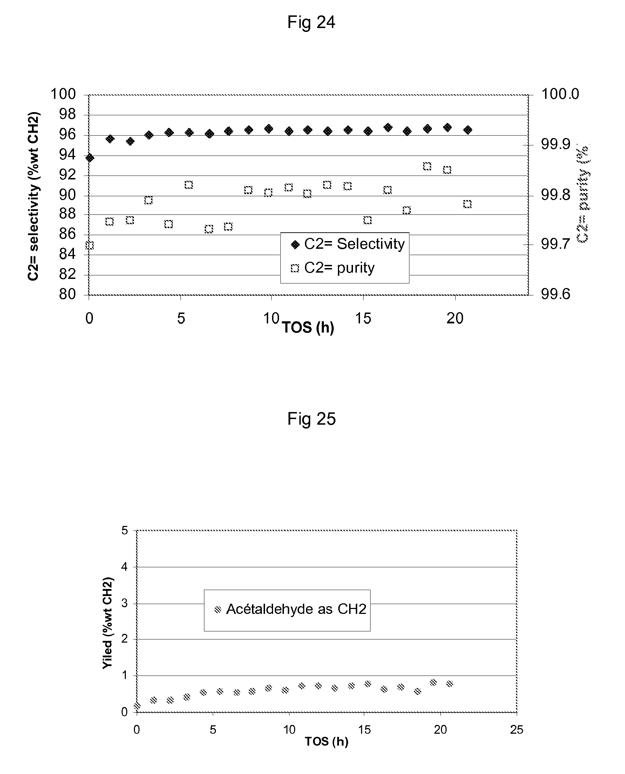

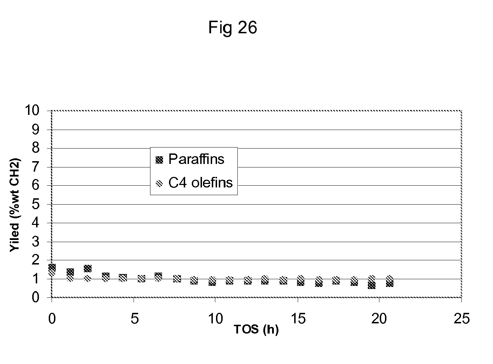

Operating conditions: For the following experiments, a pure ethanol feed has been used at 350°C, under 1.35 bara and with an ethanol space velocity of 2 h⁻¹. The results are displayed on fig 23-26.

## Claims

1. Process for the dehydratation of at least an alcohol to make at least an olefin, comprising :
introducing in a reactor a stream (A) comprising at least an alcohol, optionally water, optionally an inert component, contacting said stream with a catalyst in said reactor at conditions effective to dehydrate at least a portion of the alcohol to make an olefin,
recovering from said reactor an olefin containing stream (B), Wherein,
• the catalyst is a crystalline silicate having a ratio Si/Al of at least about 100,
• the WHSV on the alcohols is at least 4 h⁻¹,
• the temperature ranges from 280°C to 450°C.

2. Process according to claim 1 wherein the weight proportions of respectively alcohol, water and inert component are 10-100/0-20/0-70, the total being 100.

3. Process according to any one of the preceding claims wherein the pressure of the reactor ranges from 0.5 to 5 bars absolute (50 kPa to 0.5 MPa).

4. Process according to claim 3 wherein the pressure of the reactor ranges from 1.2 to 2 bars absolute (0.12 MPa to 0.2 MPa).

5. Process according to any one of the preceding claims wherein the temperature ranges from 280°C to 450°C.

6. Process according to claim 5 wherein the temperature ranges from 330°C to 380°C.

7. Process according to any one of the preceding claims wherein the WHSV ranges from 5 to 15 h⁻¹.

8. Process according to claim 7 wherein the WHSV ranges from 7 to 12 h⁻¹.

9. Process according to any one of the preceding claims wherein the alcohol is selected among ethanol, propanol, butanol and phenylethanol.

10. Process according to any one of the preceding claims wherein the inert component is selected among the saturated hydrocarbon having up to 8 carbon atoms, nitrogen and carbon dioxide.

11. Process according to claim 10 wherein the inert component is pentane.

12. Process according to any one of the preceding claims wherein the crystalline silicate is selected among the MFI and the MEL type zeolites.

13. Process according to claim 12 wherein the Si/Al ratio ranges from 100 to 1000.

14. Process according to claim 12 or 13 wherein the crystalline silicate is steamed to remove aluminium from the crystalline silicate framework.

15. Process according to claim 14 wherein, further to the steaming, aluminium is extracted from the catalyst by contacting the catalyst with a complexing agent for aluminium to remove from pores of the framework alumina deposited therein during the steaming step thereby to increase the silicon/aluminium atomic ratio of the catalyst.
